# FASCICULE DE BREVET EUROPEEN

(11) **EP 2 884 116 B1**
(45) Date de publication et mention de la délivrance du brevet: **22.01.2020**
(21) Numéro de dépôt: 14190701.4
(22) Date de dépôt: 28.10.2014
(51) Int. Cl.: F04D 29/42, A61M 16/00, F04D 29/66

(54) **Appareil d'assistance respiratoire à turbine motorisée et butées**
Gerät zur Atmungsunterstützung mit motorisierter Turbine und Endanschlägen
Breathing assistance apparatus with powered turbine and abutments

(30) Priorité: 10.12.2013 FR 1362355
(43) Date de publication de la demande: 17.06.2015
(73) Titulaire: Air Liquide Medical Systems, 92160 Antony (FR)
(72) Inventeur: Guiducci, Hadrien, 75018 Paris (FR)
(74) Mandataire: Pittis, Olivier

(56) Documents cités:
- EP-A2- 0 872 643
- WO-A1-2007/048205
- US-B2- 6 551 059

## Description

L'invention concerne un appareil d'assistance respiratoire équipé d'une turbine motorisée permettant de générer un flux de gaz, en particulier un flux d'air, comprenant des butées agencées entre la paroi supérieure du boitier interne renfermant la turbine et le pavillon surmontant ladite turbine.

Certains appareils d'assistance respiratoire mettent en œuvre une turbine motorisée, encore appelée « soufflante » ou « micro-soufflante », permettant d'aspirer de l'air ambiant et de le délivrer à débit non nul à un patient relié à l'appareil respiratoire équipé de ladite turbine. Parfois, l'air est enrichi en oxygène avant son administration par inhalation au patient. Un tel appareil d'assistance respiratoire est notamment décrit par les documents EP-A-2102504, EP-A-2122180 et WO2007/048205.

La turbine motorisée comprend habituellement un moteur électrique entrainant en rotation, via son axe ou arbre-moteur rotatif, une ou plusieurs roues à ailettes. La (ou les) roue à ailette est agencée dans un compartiment appelé « volute » dont la paroi supérieure forme couvercle recouvrant et protégeant la roue à ailettes.

La volute comporte un orifice d'entrée de gaz par lequel l'air aspiré pénètre dans la volute et un orifice de sortie qui est par ailleurs en communication fluidique avec un conduit de sortie par lequel est expulsé l'air aspiré par la roue à ailettes, pendant sa rotation.

La volute est habituellement elle-même surmontée d'un pavillon comprenant un passage d'air situé en vis-à-vis de l'orifice d'entrée de gaz de la volute de manière à créer une continuité fluidique entre ces parties.

L'assemblage moteur/roue/volute est généralement fixé au sein de l'appareil respiratoire par un système souple, tel un manchon en mousse ou analogue entourant ces parties, permettant de diminuer les vibrations transmises et le bruit généré par la turbine.

Or, le positionnement précis de cet assemblage moteur/roue/volute influe directement sur les performances de la turbine.

Ainsi, si l'espace situé au dessus de la volute et du pavillon où l'air passe avant d'entrer dans le compartiment où se trouve la roue se trouve trop diminué, suite à un choc ou du fait d'une dépression (aspiration) trop importante, la section de passage de l'air se retrouve alors trop réduite, ce qui engendre un effondrement des performances de la turbine et du débit de gaz généré.

L'insertion de plots coniques ou cylindriques, ou de mousse dans le chemin d'air dans l'espace situé au dessus de la volute pour maintenir un espacement minimum n'est pas une solution idéale car ces éléments peuvent perturber la circulation d'air et diminuer les performances de la machine.

Le problème à résoudre est d'améliorer le positionnement précis de la turbine, en particulier de l'assemblage moteur/roue/volute/pavillon, au sein du compartiment d'un appareil d'assistance respiratoire destiné à recevoir cet assemblage et de maintenir une grande section de passage de gaz entre la paroi dudit compartiment et ledit assemblage moteur/roue/volute/pavillon, même en cas de choc violent.

La solution de l'invention est un appareil d'assistance respiratoire comprenant :
- un compartiment interne comportant une paroi supérieure,
- une turbine motorisée comprenant un moteur électrique entraînant un arbre-moteur portant au moins une roue à ailettes,
- une volute surmontant ladite turbine motorisée et entourant au moins une partie de ladite au moins une roue à ailettes, ladite volute comprenant un orifice d'entrée de gaz et un orifice de sortie de gaz, typiquement d'air, la volute étant formée d'une partie inférieure de volute et d'une partie supérieure de volute assemblées l'une à l'autre de sorte de définir entre elles un logement contenant la roue à ailettes, et
- un pavillon surmontant ladite volute, ledit pavillon comprenant un passage de gaz situé en regard de l'orifice d'entrée de la volute de manière à permettre une communication fluidique entre ledit passage de gaz du pavillon et ledit orifice d'entrée de la volute, et ledit pavillon étant espacé de la paroi supérieure du compartiment interne de manière à créer entre eux un espacement de circulation du gaz, plusieurs butées ayant une forme de paroi longitudinalement rectiligne ou incurvée, étant agencées dans l'espacement entre le pavillon et la paroi supérieure du compartiment interne.

Selon le cas, l'appareil d'assistance respiratoire de l'invention peut comprendre l'une ou plusieurs des caractéristiques techniques suivantes :
- les butées ont une forme de paroi longitudinalement incurvée.
- les butées portées par la surface externe du pavillon ou par la surface interne de la paroi supérieure du compartiment interne, de préférence elles sont portées par la surface interne de la paroi supérieure du compartiment interne.
- le compartiment interne constitue un boitier interne comprenant une paroi supérieure et une paroi latérale. Il peut être directement intégré à la structure du boitier.
- les butées sont rigides, de préférence les butées sont en matériau plastique, par exemple un plastique de type acrylonitrile butadiene styrene (ABS), de type polycarbonate (PC) ou un mélange de type ABS-PC.
- il comprend de 2 à 20 butées, de préférence de 3 à 10 butées.
- les butées sont des parois agencées radialement ou approximativement radialement sur la surface interne de la paroi supérieure du compartiment interne.
- la paroi supérieure du compartiment interne a une forme de dôme.
- la paroi supérieure du compartiment interne comprend, selon un mode de réalisation particulier, une protubérance centrale se projetant coaxialement en direction de l'arbre-moteur de la turbine.
- les butées et la paroi supérieure du compartiment interne sont formées d'une seule pièce, de préférence par moulage.
- selon un autre mode de réalisation, les butées sont surmoulées sur la paroi supérieure du compartiment interne. Dans ce cas, les butées sont en matière souple, par exemple un thermoplastique élastomère (TPE) 60 Shore A.
- les butées sont portées par la surface interne de la paroi supérieure du compartiment interne et viennent prendre appui sur la surface externe du pavillon.
- les butées sont portées par la surface interne de la paroi supérieure du compartiment interne et ont un profil tridimensionnel complémentaire de la surface externe du pavillon, c'est-à-dire que le profil des butées vient épouser le profil externe du pavillon.
- les butées ont un profil tridimensionnel courbe.
- le pavillon est formé d'un matériau souple, c'est-à-dire élastique ou déformable, de préférence du silicone ou un thermoplastique élastomère (TPE) de 20 à 90 shore A.
- les butées sont conformées et orientées de manière à conférer au gaz un mouvement rotatif préalablement à l'entrée dudit gaz dans la volute.
- selon un autre mode de réalisation, les butées sont longitudinalement rectilignes, c'est-à-dire droites, de manière à redresser le gaz et éviter ou à atténuer les perturbations du gaz entrant dans la turbine.
- les butées ont une hauteur minimale H d'au moins 1 mm, de préférence d'au moins 2 mm.
- les butées ont une longueur L d'au moins 5 mm, de préférence d'au moins 10 mm.
- la volute est formée d'une partie inférieure de volute, appelée volute inférieure, et d'une partie supérieure de volute, appelée volute supérieure, assemblées l'une à l'autre de sorte de définir entre elles un logement contenant la roue à ailettes.
- une âme acoustique est agencée entre la volute et le pavillon.
- l'âme acoustique est formée d'un matériau souple ou rigide, de préférence d'un matériau souple, tel que TPE 60 Shore A.

L'invention va maintenant être décrite plus en détail en référence aux Figures annexées parmi lesquelles :
- la Figure 1 représente une vue en coupe d'un mode de réalisation d'un ensemble turbine apte à être agencé dans le compartiment interne d'un appareil d'assistance respiratoire selon la présente invention,
- la Figure 2 est une vue de dessous du dôme formant la paroi supérieure portant les butées du compartiment interne de l'appareil d'assistance respiratoire de la Figure 1,
- les Figures 3A et 3B schématisent les flux d'air sous le dôme de la Figure 2 selon deux modes de réalisation différentes des butées,
- la Figure 4 schématise les flux d'air dans l'ensemble turbine de la Figure 1,
- la Figure 5 représente une vue éclatée de l'ensemble turbine de la Figure 1,
- la Figure 6 montre l'ensemble turbine de la Figure 1 inséré dans le compartiment interne d'un appareil d'assistance respiratoire selon la présente invention,
- la Figure 7 montre l'ensemble turbine de la Figure 1 extrait du compartiment formant boitier autour dudit ensemble turbine, et
- la Figure 8 schématise une butée en vue grossie.

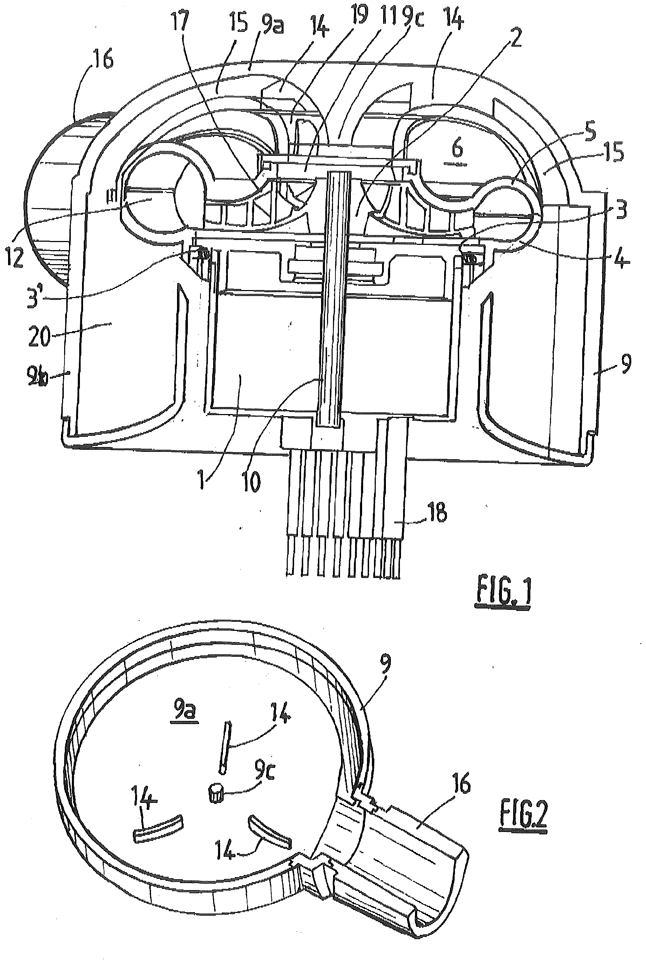

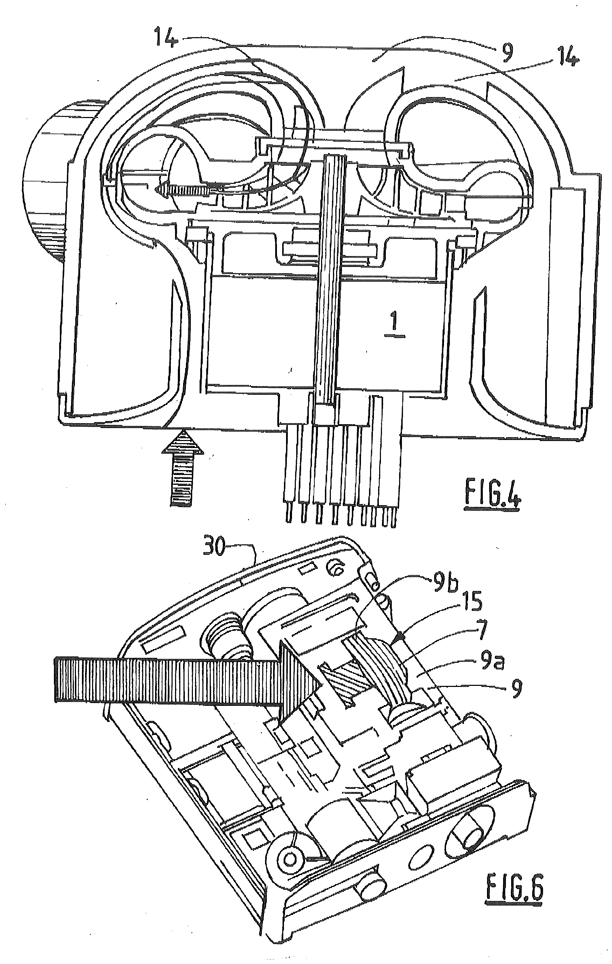

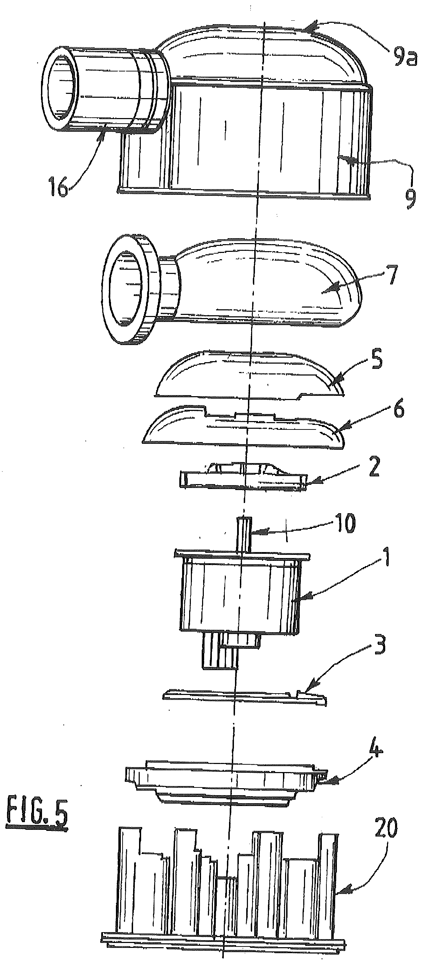

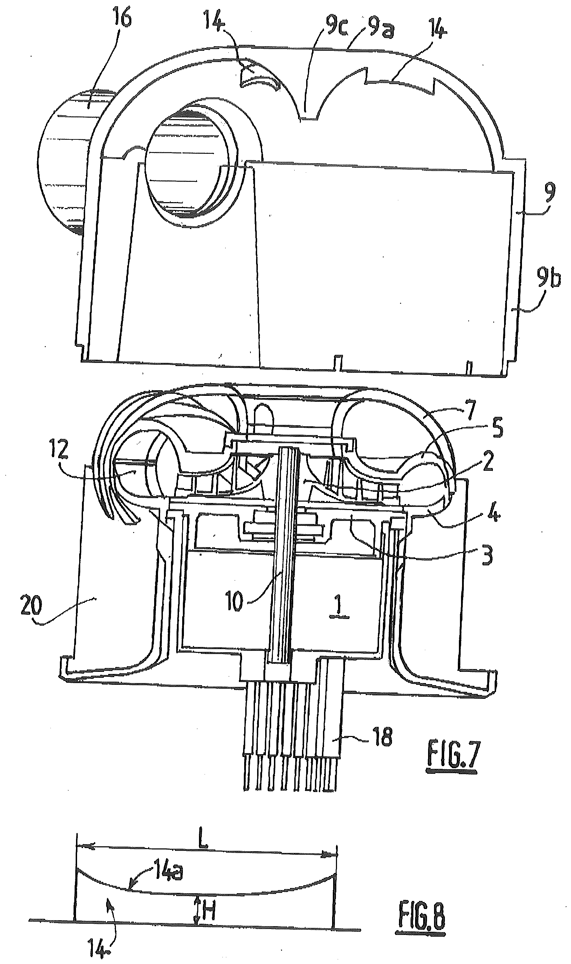

La Figure 1 représente un mode de réalisation, vu en coupe, d'un ensemble turbine 1, 2, 10 incorporé dans le compartiment interne 9 d'un appareil d'assistance respiratoire. Cet ensemble turbine est également montré extrait du compartiment 9 sur la Figure 7, en vue éclatée sur la Figure 5 et inséré dans un appareil d'assistance respiratoire 30 sur la Figure 6.

Comme on le voit, le compartiment interne 9 forme un boitier interne à l'appareil 30 au sein duquel est positionné et maintenu l'ensemble turbine comprenant la turbine 1 motorisée et ses différents éléments, comme détaillé ci-après. Le compartiment interne 9 peut constituer un boitier inclus à demeure au sein de l'appareil 30 lui-même ou être amovible, c'est-à-dire pouvant être retiré de l'appareil 30.

Plus précisément, le compartiment interne 9 comprend une paroi périphérique 9b, de forme générale cylindrique, entourant l'ensemble turbine. La paroi périphérique 9b est surmontée d'une paroi supérieure 9a, ici en forme de dôme, laquelle comprend sur la surface interne une protubérance centrale 9c se projetant en direction de l'ensemble turbine et, plus particulièrement de façon coaxiale à l'arbre 10 du moteur 1.

L'ensemble turbine comprend quant à lui une turbine motorisée 1, 10 comprenant un moteur électrique 1 entraînant un arbre-moteur 10, c'est-à-dire un axe rotatif, sur lequel est agencée une roue à ailettes 2. Selon un autre mode de réalisation, l'arbre-moteur 10 peut porter plusieurs roues 2 à ailettes agencées en parallèle sur l'axe 10. Le moteur 1 de la turbine est alimenté, de façon classique, en courant électrique par des fils électriques 18 ou analogue véhiculant un courant électrique issu d'une source de courant, tel qu'une ou plusieurs batteries, ou le secteur électrique.

La turbine motorisée 1, 10 est surmontée d'une volute 4, 5 qui forme un logement ou compartiment 17 à roue entourant la roue à ailettes 2. La volute 4, 5 est ici formée de deux parties venant s'assembler l'une à l'autre, à savoir une partie inférieure de volute 4, encore appelée volute inférieure, et une partie supérieure de volute 5, encore appelée volute supérieure, qui définissent le compartiment à roue 17 et prennent « en sandwich » la roue à ailettes 2. Ces deux parties de volute 4, 5 sont fixées de manière étanche l'une à l'autre par exemple par collage ou analogue, ou à l'aide d'une pièce supplémentaire/intermédiaire, pour éviter des fuites d'air à leur jonction.

La volute inférieure 4 est agencée en dessous de la roue 2 à ailettes, c'est-à-dire entre la roue 2 et le moteur 1 de la turbine, alors que la volute supérieure est agencée au-dessus de la roue à ailettes 2, c'est-à-dire de l'autre côté de la roue à ailettes 2 par rapport à la volute inférieure 4 ; cet agencement est illustré sur la Figure 1.

Il est prévu en outre une plaque de séparation 3 formant le fond du compartiment à roue 17 et séparant la roue 2 du moteur 1, comme visible sur la Figure 1. La plaque de séparation 3 est ici intégrée au moteur.

Par ailleurs, comme illustré en Figure 1, on prévoit aussi un espace vide sous la roue 2 entre la plaque de fond 3 et ladite roue 2 à ailettes, ainsi qu'un joint 3' annulaire, localisé sous la plaque de fond 3 surmontant le moteur 1, permettant d'assurer une étanchéité gazeuse entre le moteur 1 et la volute inférieure 4, c'est-à-dire que le joint annulaire 3' est pris en sandwich entre le moteur 1 et la volute inférieure 4.

La volute 4, 5 comprend par ailleurs un orifice d'entrée 11 de gaz par lequel entre le gaz, typiquement de l'air ou de l'air enrichi en oxygène (l'air étant enrichi en amont), qui est aspiré par la roue 2 à ailettes lors de sa rotation, et un orifice de sortie 12 de gaz par lequel est expulsé le gaz aspiré par la roue 2, typiquement de l'air ou de l'air enrichi en oxygène.

Avant de pénétrer au sein de la volute 4, 5, c'est-à-dire dans le logement 17 contenant la roue 2 à ailettes rotative, le gaz traverse un pavillon 7 qui surmonte la volute 4, 5. Ce pavillon 7 comporte un passage central 19 situé en regard de l'orifice d'entrée 11 de la volute et qui est en communication fluidique avec ledit orifice d'entrée 11 de manière à guider l'air aspiré vers l'intérieur de la volute 4, 5, c'est-à-dire vers le compartiment à roue 17.

Comme on le voit sur la Figure 1, la protubérance centrale 9c se projette au moins partiellement dans passage central 19 en direction de l'axe 10 du moteur 1. De préférence, la protubérance centrale 9c et l'axe 10 du moteur 1 sont coaxiaux ou approximativement coaxiaux.

Par ailleurs, le compartiment interne 9 comprend un conduit 16 de sortie de flux gazeux en communication fluidique avec l'orifice de sortie 12 de gaz de la volute 4, 5.

Entre le pavillon 7 et la volute supérieure 5 est agencée une âme acoustique 6 qui peut être rigide ou souple, de préférence souple, laquelle permet d'atténuer le bruit généré par la rotation de la roue 2. Par exemple, l'âme acoustique 6 peut être formée de silicone ou de thermoplastique élastomère (TPE) entre 30 et 90 Shores.

Le pavillon 7 est espacé de la paroi supérieure 9a du compartiment interne 9 formant boitier de manière à créer entre eux un espace ou espacement 15 de circulation du gaz, comme détaillé ci-après.

Par ailleurs, le moteur 1 de la turbine et préférentiellement au moins une partie de la volute inférieure 4 sont enveloppés dans un manchon 20 en silicone, TPE entre 30 et 90 Shores ou analogue permettant de réduire les émissions sonores, c'est-à-dire le bruit généré par les rotations du moteur 1 et de la roue à ailettes 2.

Dans le mode de réalisation de la Figure 1, on a également aménagé un passage de gaz le long du moteur 1 de manière à ce que le gaz, i.e. l'air, vienne balayer la carcasse externe dudit moteur 1 avant de continuer son cheminement dans l'espacement 15, puis de traverser le pavillon 7 avant d'arriver ensuite dans la volute 4, 5, comme schématisé en Figure 4.

Ceci permet de refroidir le moteur 1 en évacuant une partie des calories générées lors de ses rotations. Cette configuration n'est cependant pas obligatoire puisque le gaz pourrait arriver radialement, directement dans l'espacement 15, c'est-à-dire sans passer autour du moteur 1.

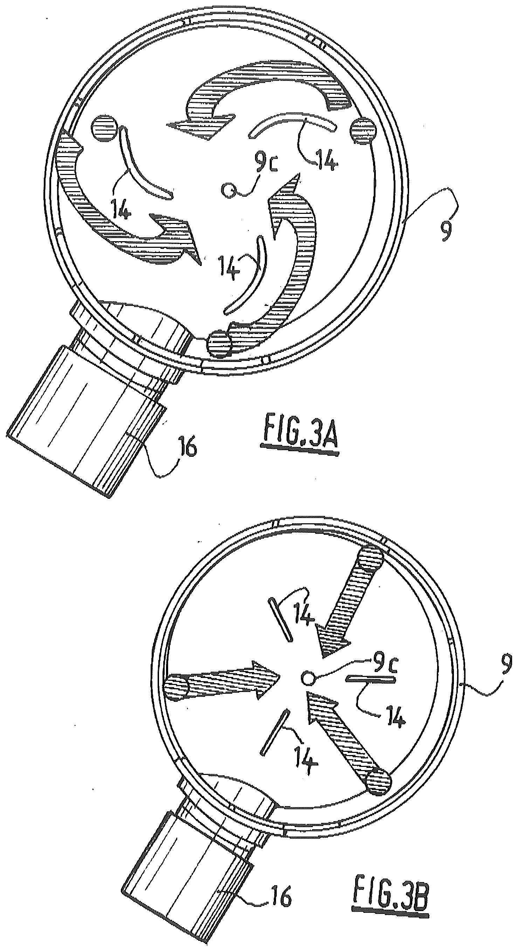

Selon la présente invention, afin de garantir un espacement 15 suffisant entre pavillon 7 et paroi supérieure 9a du compartiment interne 9, typiquement un espacement d'au moins 2 à 3 mm, sont prévues plusieurs butées 14 formées de petites parois rectilignes (Fig. 3B) ou incurvées (Fig. 3A), de préférence incurvées, lesquelles sont agencées dans l'espacement 15 entre le pavillon 7 et la paroi supérieure 9a du compartiment interne 9. De telles formes permettent d'améliorer l'écoulement et les performances de la turbine.

Comme montré sur les Figures, les butées 14 sont agencées, dans le mode de réalisation présenté ici, sur la surface interne de la paroi supérieure ou dôme 9a du compartiment 9. Toutefois, on peut également agencer tout ou partie des butées 14 directement sur la surface externe du pavillon 7.

L'incurvation des butées 14 peut être identique pour toutes les butées 14 ou certaines peuvent avoir une incurvation différente des autres. Le rayon de courbure des butées 14 incurvées, c'est-à-dire l'incurvation souhaitée, est choisi de manière à leur donner une forme aérodynamique générant le moins de pertes de charge tout en conférant au flux de gaz une pré-rotation donnée, comme schématisé en Figure 3A, avant son entrée dans le pavillon 7. En effet, une pré-rotation du flux gazeux peut être avantageuse car elle peut permettre d'améliorer les caractéristiques de débit et/ou de pression du gaz délivré par l'appareil respiratoire 30.

Plus généralement, les butées 14 incurvées (Fig. 3A) ou rectilignes (Fig. 3B) sont orientées vers le centre du dôme 9a, c'est-à-dire radialement en direction de la partie protubérance 9c qui se projette vers l'arbre-moteur 10 en étant coaxiale avec celui-ci, ce qui permet d'orienter le flux de gaz dans l'espacement 15 vers le centre du dôme formant paroi supérieure 9a du compartiment 9 de manière soit linéaire/translative (voir flèches sur Fig. 3B), soit rotative (voir flèches sur Fig. 3A).

Les butées 14 sont avantageusement formées d'une seule pièce avec la paroi supérieure 9a du compartiment ou boitier 9 interne de l'appareil respiratoire, par exemple en une pièce moulée en matériau plastique, tel que ABS, ABS-PC ou PC. On peut aussi imaginer que les butées 14 soient en matériaux souple avec une valeur Shore importante pour augmenter le découplage vibratoire tout en maintenant suffisamment de rigidité et ce, pour éviter que l'ensemble ne s'écrase.

Afin d'assurer un bon maintien en position de l'ensemble turbine au sein du compartiment 9, on prévoit au moins 2 butées 14, de préférence au moins 3 butées 14. Dans tous les cas, les butées 14 constituent des ailettes aérodynamiques rigides garantissant que l'espacement 15, c'est-à-dire la surface de passage du gaz au-dessus du pavillon 7, soit toujours au minimum égale à une valeur donnée, typiquement d'au moins 2 à 5 mm, ce qui correspond à une section de passage d'environ 1 à 30 cm².

Pour ce faire, comme illustré en Figure 8, les butées 14, qu'elles soient rectilignes ou incurvées, ont typiquement une hauteur minimale H d'au moins 2 mm et une longueur minimale L d'au moins 5 mm, de préférence d'au moins 10 mm, voire jusqu'à 30 mm environ, comme illustré en Figure 8.

Lorsque l'ensemble turbine comprenant le pavillon 7 est inséré dans le compartiment 9 d'un appareil respiratoire 30 comme illustré en Figure 6, les butées 14 portées par la surface interne de la paroi supérieure 9a du compartiment interne 9, viennent prendre appui sur la surface externe du pavillon 7, comme visible sur la Figure 1.

Afin d'améliorer le contact entre les butées 14 et le pavillon 7, les butées 14 sont conformées de manière à présenter un profil tridimensionnel complémentaire de la surface externe du pavillon 7. En d'autres termes, la surface de contact 14a des butées 14 qui vient en contact mécanique contre la surface externe du pavillon 7, présentent un profil complémentaire, en particulier une courbure, de celui du pavillon 7 de manière à venir épouser les contours externes dudit pavillon 7 à l'endroit où les butées viennent prendre appui sur le pavillon 7.

Avantageusement, le pavillon 7 est en un matériau souple, c'est-à-dire déformable et/ou élastique, tel que TPE ou silicone. Ceci permet d'éviter, en cas de choc axial, un écrasement des butées 14 rigides contre le pavillon 7 puisque tout choc sera amorti par le pavillon 7 souple qui joue alors un rôle d'amortisseur des chocs orientés dans l'axe du moteur 1 mais aussi des chocs radiaux.

En fait, le fait de mettre en œuvre des butées tridimensionnelles selon l'invention fait qu'une partie plus ou moins importante des efforts radiaux peut être reprise.

Un appareil d'assistance respiratoire 30 selon la présente invention est utilisable pour traiter diverses pathologies respiratoires, notamment les broncho-pneumopathie chronique obstructive (BPCO), syndrome de détresse respiratoire aiguë (SDRA), syndrome obésité-hypoventilation (SOH), syndrome d'apnées obstructives du sommeil (SAOS), maladies neuromusculaires, mucoviscidose, déformations thoraciques, respiration de Cheyne-Stokes, la tuberculose, les œdème pulmonaire etc.

## Revendications

1. Appareil d'assistance respiratoire comprenant :
- un compartiment interne (9) comportant une paroi supérieure (9a),
- une turbine motorisée (1, 10) comprenant un moteur électrique (1) entraînant un arbre-moteur (10) portant au moins une roue à ailettes (2),
- une volute (4, 5) surmontant ladite turbine motorisée (1, 10) et entourant au moins une partie de ladite au moins une roue à ailettes (2), ladite volute (4, 5) comprenant un orifice d'entrée (11) de gaz et un orifice de sortie (12) de gaz, la volute (4, 5) étant formée d'une partie inférieure de volute (4) et d'une partie supérieure de volute (5) assemblées l'une à l'autre de sorte de définir entre elles un logement (17) contenant la roue à ailettes (2), et **caractérisé par** un pavillon (7) surmontant ladite volute (4, 5), ledit pavillon (7) comprenant un passage de gaz (19) situé en regard de l'orifice d'entrée (11) de la volute (4, 5) de manière permettre une communication fluidique entre ledit passage de gaz (19) du pavillon (7) et ledit orifice d'entrée (11) de la volute (4, 5), et ledit pavillon (7) étant espacé de la paroi supérieure (9a) du compartiment interne (9) de manière à créer entre eux un espacement (15) de circulation du gaz, plusieurs butées (14) ayant une forme de paroi longitudinalement rectiligne ou incurvée, étant agencées dans l'espacement (15) entre le pavillon (7) et la paroi supérieure (9a) du compartiment interne (9).

2. Appareil selon la revendication précédente, **caractérisé en ce que** les butées (14) sont portées par la surface externe du pavillon (7) ou par la surface interne de la paroi supérieure (9a) du compartiment interne (9), de préférence par la surface interne de la paroi supérieure (9a) du compartiment interne (9).

3. Appareil selon l'une des revendications précédentes, **caractérisé en ce que** les butées (14) sont rigides, de préférence les butées (14) sont en matériau plastique.

4. Appareil selon l'une des revendications précédentes, **caractérisé en ce qu'**il comprend de 2 à 20 butées (14), de préférence de 3 à 10 butées (14).

5. Appareil selon l'une des revendications précédentes, **caractérisé en ce que** les butées (14) sont des parois agencées radialement ou approximativement radialement sur la surface interne de la paroi supérieure (9a) du compartiment interne (9).

6. Appareil selon l'une des revendications précédentes, **caractérisé en ce que** la paroi supérieure (9a) du compartiment interne (9) a une forme de dôme.

7. Appareil selon l'une des revendications précédentes, **caractérisé en ce que** les butées (14) et la paroi supérieure (9a) du compartiment interne (9) sont formés d'une seule pièce, de préférence par moulage.

8. Appareil selon l'une des revendications précédentes, **caractérisé en ce que** les butées (14) sont portés par la surface interne de la paroi supérieure (9a) du compartiment interne (9) et viennent prendre appui sur la surface externe du pavillon (7).

9. Appareil selon l'une des revendications précédentes, **caractérisé en ce que** les butées (14) sont portées par la surface interne de la paroi supérieure (9a) du compartiment interne (9) et ont un profil tridimensionnel complémentaire de la surface externe du pavillon (7).

10. Appareil selon l'une des revendications précédentes, **caractérisé en ce que** les butées (14) ont un profil tridimensionnel courbe.

11. Appareil selon l'une des revendications précédentes, **caractérisé en ce que** le pavillon (7) est formé d'un matériau souple, de préférence du silicone ou du TPE

12. Appareil selon l'une des revendications précédentes, **caractérisé en ce que** les butées (14) sont conformées et orientées de manière à conférer au gaz un mouvement rotatif préalablement à l'entrée dudit gaz dans la volute (4, 5).

13. Appareil selon l'une des revendications précédentes, **caractérisé en ce que** les butées (14) ont une hauteur minimale (H) d'au moins 2 mm, de préférence d'au moins 4 mm.

14. Appareil selon l'une des revendications précédentes, **caractérisé en ce que** les butées (14) ont une longueur (L) d'au moins 5 mm, de préférence d'au moins 10 mm.

## Patentansprüche

1. Einrichtung zur Atmungsunterstützung, umfassend:
- ein inneres Fach (9), das eine obere Wand (9a) umfasst,
- eine motorisierte Turbine (1, 10), die einen Elektromotor (1) umfasst, der eine Motorwelle (10) antreibt, die mindestens ein Flügelrad (2) trägt,
- eine Spirale (4, 5), welche die motorisierte Turbine (1, 10) überragt und mindestens einen Teil des mindestens einen Flügelrads (2) umgibt, wobei die Spirale (4, 5) eine Gaseinlassöffnung (11) und eine Gasauslassöffnung (12) umfasst, wobei die Spirale (4, 5) aus einem unteren Spiralenteil (4) und einem oberen Spiralenteil (5) gebildet ist, die so miteinander verbunden sind, dass sie zwischen sich eine Aufnahme (17) definieren, die das Flügelrad (2) enthält, und
**gekennzeichnet durch**
einen Pavillon (7), der die Spirale (4, 5) überragt, wobei der Pavillon (7) einen Gasdurchlass (19) umfasst, der sich gegenüber der Einlassöffnung (11) der Spirale (4, 5) befindet, um eine fluidische Verbindung zwischen dem Gasdurchlass (19) des Pavillons (7) und der Einlassöffnung (11) der Spirale (4, 5) zu ermöglichen, und wobei der Pavillon (7) von der oberen Wand (9a) des inneren Fachs (9) so beabstandet ist, um einen Zwischenraum (15) zur Zirkulation des Gases zwischen ihnen zu erzeugen,
mehrere Anschläge (14), die eine in Längsrichtung geradlinige oder gekrümmte Wandform aufweisen, die in dem Zwischenraum (15) zwischen dem Pavillon (7) und der oberen Wand (9a) des inneren Fachs (9) angeordnet sind.

2. Einrichtung nach dem vorstehenden Anspruch, **dadurch gekennzeichnet, dass** die Anschläge (14) von der äußeren Oberfläche des Pavillons (7) oder von der inneren Oberfläche der oberen Wand (9a) des inneren Fachs (9), vorzugsweise von der inneren Oberfläche der oberen Wand (9a) des inneren Fachs (9), getragen werden.

3. Einrichtung nach einem der vorstehenden Ansprüche, **dadurch gekennzeichnet, dass** die Anschläge (14) starr sind, die Anschläge (14) vorzugsweise aus Kunststoffmaterial bestehen.

4. Einrichtung nach einem der vorstehenden Ansprüche, **dadurch gekennzeichnet, dass** sie 2 bis 20 Anschläge (14), vorzugsweise 3 bis 10 Anschläge (14) umfasst.

5. Einrichtung nach einem der vorstehenden Ansprüche, **dadurch gekennzeichnet, dass** die Anschläge (14) radial oder annähernd radial auf der inneren Oberfläche der oberen Wand (9a) des inneren Fachs (9) angeordnete Wände sind.

6. Einrichtung nach einem der vorstehenden Ansprüche, **dadurch gekennzeichnet, dass** die obere Wand (9a) des inneren Fachs (9) eine Kuppelform aufweist.

7. Einrichtung nach einem der vorstehenden Ansprüche, **dadurch gekennzeichnet, dass** die Anschläge (14) und die obere Wand (9a) des inneren Fachs (9) in einem Stück, vorzugsweise durch Formguss, gebildet sind.

8. Einrichtung nach einem der vorstehenden Ansprüche, **dadurch gekennzeichnet, dass** die Anschläge (14) von der inneren Oberfläche der oberen Wand (9a) des inneren Fachs (9) getragen werden und auf der äußeren Oberfläche des Pavillons (7) anliegen.

9. Einrichtung nach einem der vorstehenden Ansprüche, **dadurch gekennzeichnet, dass** die Anschläge (14) von der inneren Oberfläche der oberen Wand (9a) des inneren Fachs (9) getragen werden und ein dreidimensionales Profil aufweisen, das zu der äußeren Oberfläche des Pavillons (7) komplementär ist.

10. Einrichtung nach einem der vorstehenden Ansprüche, **dadurch gekennzeichnet, dass** die Anschläge (14) ein gekrümmtes dreidimensionales Profil aufweisen.

11. Einrichtung nach einem der vorstehenden Ansprüche, **dadurch gekennzeichnet, dass** der Pavillon (7) aus einem flexiblen Material, vorzugsweise Silikon oder TPE, gebildet ist.

12. Einrichtung nach einem der vorstehenden Ansprüche, **dadurch gekennzeichnet, dass** die Anschläge (14) so geformt und ausgerichtet sind, um dem Gas vor dem Eintreten des Gases in die Spirale (4, 5) eine Drehbewegung zu verleihen.

13. Einrichtung nach einem der vorstehenden Ansprüche, **dadurch gekennzeichnet, dass** die Anschläge (14) eine Mindesthöhe (H) von mindestens 2 mm, vorzugsweise von mindestens 4 mm aufweisen.

14. Einrichtung nach einem der vorstehenden Ansprüche, **dadurch gekennzeichnet, dass** die Anschläge (14) eine Länge (L) von mindestens 5 mm, vorzugsweise von mindestens 10 mm aufweisen.

## Claims

1. Breathing assistance appliance comprising:
- an inner compartment (9) comprising an upper wall (9a),
- a motorised turbine (1, 10) comprising an electric motor (1) driving a motor shaft (10) carrying at least one impeller wheel (2),
- a volute (4, 5) surmounting said motorised turbine (1, 10) and surrounding at least one portion of said at least one impeller wheel (2), said volute (4, 5) comprising a gas inlet orifice (11) and a gas outlet orifice (12), the volute (4, 5) being formed of a lower volute (4) portion and an upper volute (5) portion assembled together so as to define between them, a housing (17) containing the impeller wheel (2), and
**characterised by** a pavilion (7) surmounting said volute (4, 5), said pavilion (7) comprising a gas passage (19) situated opposite the inlet orifice (11) of the volute (4, 5) so as to make it possible for a fluidic communication between said gas passage (19) of the pavilion (7) and said inlet orifice (11) of the volute (4, 5), and said pavilion (7) being spaced from the upper wall (9a) of the inner compartment (9) so as to create between them, a space (15) for circulating gas,
several stops (14) having a longitudinally rectilinear or curved-shaped wall, being arranged in the space (15) between the pavilion (7) and the upper wall (9a) of the inner compartment (9).

2. Appliance according to the preceding claim, **characterised in that** the stops (14) are carried by the outer surface of the pavilion (7) or by the inner surface of the upper wall (9a) of the inner compartment (9), preferably by the inner surface of the upper wall (9a) of the inner compartment (9).

3. Appliance according to one of the preceding claims, **characterised in that** the stops (14) are rigid, preferably the stops (14) are made of plastic material.

4. Appliance according to one of the preceding claims, **characterised in that** it comprises 2 to 20 stops (14), preferably 3 to 10 stops (14).

5. Appliance according to one of the preceding claims, **characterised in that** the stops (14) are walls radially or approximately radially arranged over the inner surface of the upper wall (9a) of the inner compartment (9).

6. Appliance according to one of the preceding claims, **characterised in that** the upper wall (9a) of the inner compartment (9) has a dome shape.

7. Appliance according to one of the preceding claims, **characterised in that** the stops (14) and the upper wall (9a) of the inner compartment (9) are formed of one single part, preferably by moulding.

8. Appliance according to one of the preceding claims, **characterised in that** the stops (14) are carried by the inner surface of the upper wall (9a) of the inner compartment (9) and bear on the outer surface of the pavilion (7).

9. Appliance according to one of the preceding claims, **characterised in that** the stops (14) are carried by the inner surface of the upper wall (9a) of the inner compartment (9) and have a complementary three-dimensional profile of the outer surface of the pavilion (7).

10. Appliance according to one of the preceding claims, **characterised in that** the stops (14) have a curved three-dimensional profile.

11. Appliance according to one of the preceding claims, **characterised in that** the pavilion (7) is formed of a flexible material, preferably made of silicone or of TPE.

12. Appliance according to one of the preceding claims, **characterised in that** the stops (14) are shaped and oriented so as to confer to the gas, a rotating movement prior to the entry of said gas into the volute (4, 5).

13. Appliance according to one of the preceding claims, **characterised in that** the stops (14) have a minimum height (H) of at least 2mm, preferably of at least 4mm.

14. Appliance according to one of the preceding claims, **characterised in that** the stops (14) have a length (L) of at least 5mm, preferably of at least 10mm.
